# EUROPEAN PATENT SPECIFICATION

(11) **EP 2 977 451 B1**
(45) Date of publication and mention of the grant of the patent: **21.11.2018**
(21) Application number: 14770623.8
(22) Date of filing: 03.02.2014
(51) Int. Cl.: C12N 7/01, A61K 39/12, G01N 33/53

(54) **NOVEL KOREAN-TYPE PORCINE REPRODUCTIVE AND RESPIRATORY SYNDROME (PRRS) VIRUS**
NEUARTIGES VIRUS DES KOREANISCHEN REPRODUKTIONS- UND ATEMWEGSSYNDROM DER SCHWEINE (PRRS)
NOUVEAU VIRUS DU SYNDROME DYSGÉNÉSIQUE ET RESPIRATOIRE PORCIN (SDRP) DE TYPE CORÉEN

(30) Priority: 20.03.2013 KR 20130029891
(43) Date of publication of application: 27.01.2016
(73) Proprietor: Optipharm Co., Ltd, Cheongwon-gun, Chungcheongbuk-do 363-954 (KR)
(72) Inventor: KIM, Hyun Il, Seoul 136-055 (KR); SHIN, Seong Ho, Cheongwon-gun Chungcheongbuk-do 363-951 (KR); HAN, Beom Ku, Daejeon 302-734 (KR)
(74) Representative: Icosa
(86) International application number: PCT/KR2014/000897
(87) International publication number: WO 2014/148738

(56) References cited:
- WO-A2-2011/153351
- KR-A- 20070 086 084
- KR-A- 20110 052 183
- KR-A- 20120 026 436
- KR-A- 20120 131 533
- KR-A- 20120 134 015
- KR-A- 20130 010 426
- KR-B1- 100 241 221
- KR-B1- 101 191 518
- EUN-JIN CHOI ET AL: "Genetic diversity of porcine reproductive and respiratory syndrome virus in Korea", JOURNAL OF VETERINARY SCIENCE, vol. 14, no. 2, 1 January 2013 (2013-01-01), page 115, XP055303265, KR ISSN: 1229-845X, DOI: 10.4142/jvs.2013.14.2.115
- CHULSEUNG LEE ET AL: "Prevalence and phylogenetic analysis of the isolated type I porcine reproductive and respiratory syndrome virus from 2007 to 2008 in Korea", VIRUS GENES, KLUWER ACADEMIC PUBLISHERS, BO, vol. 40, no. 2, 9 January 2010 (2010-01-09), pages 225-230, XP019775258, ISSN: 1572-994X, DOI: 10.1007/S11262-009-0433-3
- EEURI NAM ET AL: "Complete genomic characterization of a European type 1 porcine reproductive and respiratory syndrome virus isolate in Korea", ARCHIVES OF VIROLOGY ; OFFICIAL JOURNAL OF THE VIROLOGY DIVISIONOF THE INTERNATIONAL UNION OF MICROBIOLOGICAL SOCIETIES, SPRINGER-VERLAG, VI, vol. 154, no. 4, 19 March 2009 (2009-03-19), pages 629-638, XP019722908, ISSN: 1432-8798
- YOON S H ET AL: "Genetic characterization of the Korean porcine reproductive and respiratory syndrome viruses based on the nucleocapsid protein gene (ORF7) sequences", ARCHIVES OF VIROLOGY ; OFFICIAL JOURNAL OF THE VIROLOGY DIVISIONOF THE INTERNATIONAL UNION OF MICROBIOLOGICAL SOCIETIES, SPRINGER-VERLAG, VI, vol. 153, no. 4, 29 January 2008 (2008-01-29), pages 627-635, XP019592518, ISSN: 1432-8798
- CHA S H ET AL: "Molecular characterization of recent Korean porcine reproductive and respiratory syndrome (PRRS) viruses and comparison to other Asian PRRS viruses", VETERINARY MICROBIOLOGY, ELSEVIER BV, NL, vol. 117, no. 2-4, 31 October 2006 (2006-10-31), pages 248-257, XP024935684, ISSN: 0378-1135, DOI: 10.1016/J.VETMIC.2006.05.007 [retrieved on 2006-10-31]

## Description

### FIELD OF THE INVENTION

The present invention relates to a Korean-type porcine reproductive and respiratory syndrome virus (PRRSV) and a vaccine composition using the same, and the virus for use in preventing porcine reproductive and respiratory syndrome.

### DESCRIPTION OF THE RELATED ART

In 1987, the occurrence of a novel porcine disease from an unknown cause began to be reported around the swine raising region in U.S.A. This disease, in which reproductive disorders and respiratory infections occur together, explosively increased in summer in 1988 and 1989. Initially, this disease, which suddenly emerged and caused massive damages to the US swine industry, was called mystery swine disease. Thereafter, since the causative virus has been isolated and the clinical symptoms, pathogenicity, etc. have been studied, this disease is now internationally named PRRS (Porcine Reproductive and Respiratory Syndrome) according to the characteristics of the clinical symptoms which express both reproductive disorders and respiratory infections.

In Korea, although no prevalence of symptoms similar to this disease has been reported, this disease has been studied since 1992. A diagnostic method thereof has been established, and epidemiological investigation for Korean swine farms has been conducted. As a result, it was confirmed that this disease was also introduced in Korea, and the causative virus was isolated from Korean infected farms. In Asia, the confirmation of the occurrence and the isolation of the causative virus were reported in Japan and Taiwan in addition to Korea.

Even after the report of the occurrence, the exact cause was unknown, so the swine industry got into great confusion. In June, 1991, Dr. Wensvoort at the Netherlands Central Veterinary Laboratory isolated the causative virus using porcine alveolar macrophage for the first time. This virus was named Lelystad virus according to the place name where the Netherlands Central Veterinary Laboratory was located, and soon afterwards, the causative virus was isolated in many countries including U.S.A. As a result of the investigation on its properties and pathogenicity, etc., it was discovered that the new porcine disease, which has resulted in both reproductive disorders and respiratory infections in U.S.A. and Europe until then, was caused by this virus.

PRRS virus, which is often called Lelystad virus in Europe and SIRS virus in U.S.A, has an outer membrane, and this virus is a small globular virus having a diameter of 45-80mm, and there are small protrusions on its surface. The genes are located on a ssRNA having a diameter of 25-35 nm. Since this virus is not so strong in an external environment, at an acidity of not more than 5 or not less than 7, the virus infection titer is reduced by 90% or more, and after 10 to 24 hours at 37°C and after 6 days at 20°C, the infection titer is reduced more than 10 times. When pigs are infected with this virus, outcomes are very various, including cases where there are no clinical symptoms and no economic damages, so that the infection can be detected only by a serum test, and serious cases enough to cause 20% of losses in the farm production.

As can be known from the disease name, reproductive disorders and respiratory diseases occur together. The characteristics of reproductive disorders caused by PRRS virus infection include late miscarriages, which mainly occur between 107 days and 113 days of pregnancy, or premature farrowings, the occurrence of stillborn and mummified pigs, sudden increase in mortality rate before weaning, increase in mortality rate after weaning, delayed return to estrus, etc. These symptoms intensively appear in infected sows and pigs born from the sows. It has been known that reproductive disorders caused by PRRS virus infection almost return to the pre-infection state after about 6 months from the early stage of the infection. Respiratory diseases caused by PRRS virus infection may appear in all ages, and in particular, the diseases intensively appear in suckling pigs and weaning pigs. In this case, fast abdominal breathing is observed, and palpebral edema, conjunctivitis, sneeze, diarrhea, etc. are observed, there are cases where body temperature increases for a short period, and neurological sign and symptom may appear, although rare. The respiratory diseases are mainly caused by a secondary infection and a complex infection with bacterial, viral pathogenic organisms, rather than by an infection with the virus alone, and in this case, pathogenicity is much higher. Other than the above, there are cases where locus caeruleus may occur on ears, abdomen, pudenda, etc., so in the United Kingdom, this disease is also called blue ear disease.

The aforementioned clinical symptoms can be readily observed when the disease proceeds acutely, whereas distinct and characteristic clinical symptoms can be rarely observed when the disease is caused by a subclinical infection or proceeds chronically. Chronic infections mainly spread in weaning pigs and at the growing-fattening stage, and respiratory systems which receive attacks by PRRS virus are infected with germs and viruses, which results in rhinitis and pneumonia. Consequently, the average daily weight gain is lowered, the feed conversion rate increases much more, and the mortality rate after weaning increases twice as compared to the average mortality rate before the infection with PRRS virus. Lung symptoms from a respiratory disease caused by PRRS virus display the pathological characteristics of pneumonitis.

PRRS virus is currently classified into Lelystad virus (Wensvoort *et al.,* 1991), which was first isolated in Europe, and VR-2332[0004] virus (Benfield *et al.,* 1992), which was isolated in North America. They are the prototypes of the European strains and the North American strains, respectively, and their sequence homology is about 50 to 70%, which is very low (Gagnon and Dea, 1998; Kwang *et al.,* 1994; Murtaugh *et al.,* 1995). Currently, PRRS virus, together with equine arthritis virus (EAV), lactate dehydrogenase-elevating virus (LDV) and simian hemorrhagic fever virus (SHFV), belongs to the order *Nidovirales* and family *Arteriviridae* (Cavanagh, 1997). PRRS virus, which is a very small enveloped virus having phosphatides, has a single-stranded RNA genome with about 15kb of positive-polarity in the regular polyhedron shaped nucleocapsid (Collins *et al.,* 1992). Genes encoding replicase, which is a nonstructural protein consisting of enzymes necessary for autoreplication of virus, occupy about 80% of the genomic RNA and are located at the 5'-terminal, and they are formed by ORFla and ORFlb having an overlapping region expressed from the genomic RNA. Out of them, ORFlb has been reported to be expressed by the ribosomal frameshift mechanism (Brierley *et al.,* 1989). Genes encoding viral structural proteins occupy about 20% of the genomic RNA and are located at the 3'-terminal. The structural proteins are expressed from 7 genes, i.e. ORF2a and ORF2b to ORF7. ORF2, ORF3 and ORF4, generate glycosylated membrane proteins which are GP2, GP3 and GP4, respectively. ORF5 generates glycoprotein GP5 envelope protein, which plays the most important role in PRRS virus neutralization activity. ORF6 generates M (matrix), and ORF7 generates N (nucleocapsid) (Meulenberg *et al.,* 1995; Bastos *et al.,* 2004). They are expressed from 5'-terminal of monocistronic subgenomic mRNA in infected cells (Meulenberg *et al.,* 1993; Synder *et al.,* 1999). Study results on the function and molecular biological characteristics of most of the proteins of PRRS virus, including nonstructural proteins as well as structural proteins, are still insufficient. When pigs are infected with PRRS virus, serious reproductive disorders and respiratory disorders are expressed as clinical symptoms. Despite the fact that it cannot be proven that the European strains and North American strains of PRRS virus are distinctly different in symptoms of the diseases, they have distinct antigenic or genotype differences (Allende *et al.,* 1999; Halbur *et al.,* 1995; Wootton *et al.,* 1998). In addition, even in each of the North American strains and the European strains, there is a difference between isolated strains. For example, it has been reported that variants of base sequences among several isolated strains belonging to the North American strains may be caused by an essential error of RNA polymerase or RNA recombination, and genetic variants thereof may lead to an important difference in pathogenicity of virus (Andreyev *et al.,* 1997; Halbur *et al.,* 1996; Ward *et al.,* 1988).

Korean patent application KR10-2013-0010426 discloses a vaccine composition for preventing Korean porcine reproductive and respiratory syndrome, comprising an attenuated or dead Korean-type PRRSV strain deposited under number KCTC11954BP. However, a vaccine suitable for Korean variants by isolating Korean-type PRRSV and the preparation method of Korean-type PRRSV using the same have not been commercialized yet.

Therefore, further studies on vaccines suitable for Korean-type PRRSV are needed.

### SUMMARY OF THE INVENTION

The present inventors conducted the studies by isolating a porcine reproductive and respiratory syndrome virus (hereinafter, PRRSV) from Korean pigs and found that the isolated virus was a novel Korean-type PRRSV, and then completed the present invention.

The object of the present invention is to provide a novel Korean-type PRRSV and a vaccine composition using the same. The object of the present invention is to provide a novel Korean-type PRRSV and a vaccine composition using the same, for use in preventing porcine reproductive and respiratory syndrome.

In order to achieve the above object, the present invention provides a Korean-type porcine reproductive and respiratory syndrome virus (hereinafter, PRRSV) (Accession Number KCTC 12096BP).

In addition, the present invention provides a vaccine composition comprising a Korean-type porcine reproductive and respiratory syndrome virus (Accession Number KCTC 12096BP) as an effective ingredient.

In addition, the present invention provides the virus vaccine composition for use in preventing porcine reproductive and respiratory syndrome, wherein the virus vaccine composition is to be administered to a pig.

In addition, the present invention provides a diagnosis kit for a Korean-type porcine reproductive and respiratory syndrome virus, comprising a Korean-type porcine reproductive and respiratory syndrome virus (Accession Number KCTC 12096BP).

In addition, the present invention provides an *in vitro* method for detecting a Korean-type porcine reproductive and respiratory syndrome virus, characterized by detecting a Korean-type porcine reproductive and respiratory syndrome virus (Accession Number KCTC 12096BP) in a cell to be infected or being infected through an antigen-antibody reaction using a Korean-type porcine reproductive and respiratory syndrome virus (Accession Number KCTC 12096BP).

The Korean-type porcine reproductive and respiratory syndrome virus with Accession Number KCTC 12096BP according to the present invention is a Korean-type porcine reproductive and respiratory syndrome virus distinguished from the European strains and the North American strains. It allows to produce a vaccine composition specific to the Korean-type porcine reproductive and respiratory syndrome virus, and which is capable of preventing the Korean-type porcine reproductive and respiratory syndrome or specifically diagnosing the infection with Korean-type porcine reproductive and respiratory syndrome virus.

### BRIEF DESCRIPTION OF DRAWINGS

**FIG. 1** is a diagram showing porcine alveolar macrophage isolated from a germ-free pig (2×10⁸ cells/ml).
**FIG. 2** is a diagram showing the results of determination on the infection with virus by the indirect immunofluorescence assay after inoculation of a porcine alveolar macrophage with a porcine reproductive and respiratory syndrome virus (hereinafter, PRRSV).
**FIG. 3** is a phylogenetic analysis diagram using the analysis results of base sequences of ORF5 of Korean-type PRRSV (JW-PRRSV).
**FIG. 4** is a diagram showing the results of measuring the antibody titer after inoculation with isolated Korean-type PRRSV (JW-PRRSV) (passage 5, passage 91).
**FIG. 5** is a diagram showing the results of measuring the antibody titer in blood after inoculation with Korean-type PRRSV (JW-PRRSV) (passage 3, passage 120).
**FIG. 6** is a diagram showing the results of measuring leukocyte and lymphocyte values in the blood of subjects after inoculation with Korean-type PRRSV (JW-PRRSV) (passage 3, passage 120).
**FIG. 7** is a diagram showing the results of analyzing clinical symptoms of subjects after inoculation with Korean-type PRRSV (JW-PRRSV) (passage 3, passage 120).
**FIG. 8** is a diagram showing the results of assessing viral shedding through PCR after inoculation with Korean-type PRRSV (JW-PRRSV) (passage 3, passage 120) (Group A: Group inoculated with MLV commercial vaccine, Group B: Group inoculated with passage 3 JW-PRRSV, Group C: Group inoculated with passage 120 JW-PRRSV, Group D: positive control, Group E: negative control).

### BEST MODE FOR CARRYING OUT THE INVENTION

The present invention provides a Korean-type porcine reproductive and respiratory syndrome virus (hereinafter, PRRSV) (Accession Number KCTC 12096BP).

The Korean-type porcine reproductive and respiratory syndrome virus (hereinafter, JW-PRRSV) (Accession Number KCTC 12096BP), which is isolated from outdoor farms, is capable of proliferation in the monkey kidney cell, MARC-145 cell, or a porcine alveolar macrophage (PAM) which is directly isolated from pigs and cultured, although not limited thereto.

In addition, the present invention provides a vaccine composition including JW-PRRSV (Accession Number KCTC 12096BP) as an effective ingredient.

The vaccine composition may include JW-PRRSV which is obtained by culturing JW-PRRSV for 3 to 120 passages. The number of passages is not limited if the activity of this virus can be maintained, but the vaccine composition may preferably include, as an effective ingredient, JW-PRRSV which is obtained by culturing JW-PRRSV for 3 passages, 5 passages, 91 passages or 120 passages.

The Korean-type porcine reproductive and respiratory syndrome virus (JW-PRRSV) according to the present invention can induce the virus protection equivalent to commercial MLV vaccine, as shown by the formation of the antibody titer and analysis of clinical symptoms. Therefore, this virus may effectively be a novel vaccine candidate substance against the porcine reproductive and respiratory syndrome virus. Further, the Korean-type porcine reproductive and respiratory syndrome virus (JW-PRRSV) according to the present invention does not lead to an inflammatory response in individuals when inoculated and exhibits safety with no side effects such as suppuration, necrosis, fever, etc. in an injection site. Also, as the viral shedding time is reduced as compared to existing PRRSV vaccines, the problem of the existing PRRSV vaccines can be improved. Therefore, this virus can be useful as a novel vaccine composition for a Korean-type porcine reproductive and respiratory syndrome virus.

The "vaccine" is a veterinary vaccine including an antigen substance. This vaccine is specific to the porcine reproductive and respiratory syndrome and administered for the purpose of inducing specific active or passive immunity.

The vaccine composition may include one or more suitable adjuvants, excipients or carriers, in addition to an effective ingredient, JW-PRRSV (Accession Number KCTC 12096BP).

Additional ingredients for enhancing an immune reaction may be adjuvants, for example, aluminum hydroxide, mineral oil or other oil, or other enhancing molecules which are added to a vaccine or are generated by the body after induction by these additional ingredients, for example, but not limited to, interferons, interleukins or growth factors.

Adjuvants used in the present invention include a substance which enhances an immune reaction of an injected animal. A variety of different adjuvants are known in this technical field. Examples of adjuvants used in the present invention may include Freund's complete and incomplete adjuvants, vitamin E, nonionic blocking polymers, muramyl dipeptide, Quil A, mineral oil and non-mineral oil, and carbopol. In a preferred embodiment, the vaccine according to the present invention may include a water-in-oil emulsion adjuvant.

Suitable carriers for the vaccine are well known to those skilled in the art and include but are not limited to proteins, sugars, etc. The carriers may be aqueous or non-aqueous solutions, suspensions, or emulsions. Examples of non-aqueous carriers are propylene glycol, polyethylene glycol, vegetable oils such as olive oil, and injectable organic esters such as ethyl oleate. Aqueous carriers include water, alcoholic/aqueous solutions, emulsions or suspensions which include saline and buffered media. Parenteral carriers include sodium chloride solution, Ringer's dextrose, dextrose and sodium chloride, lactated Ringer's or fixed oils. Intravenous carriers include fluid and nutrient replenishers, electrolyte replenishers such as those based on Ringer's dextrose, and the like. Preservatives and other additives may also be present, such as, for example antimicrobials, antioxidants, chelating agents, inert gases and the like. Preferred preservatives include formalin, thimerosal, neomycin, polymyxin B and amphotericin B.

In addition, the vaccine according to the present invention may include one or more suitable emulsifiers, such as Span or Tween.

In addition, the vaccine composition according to the present invention may include protective agents, and any protective agents known in this technical field can be used without limitation. Protective agents may preferably lactose (LPGG) or trehalose (TPGG), and more preferably trehalose.

In addition, the present invention provides a JW-PRRSV (accession number 12096BP) vaccine composition for use in preventing porcine reproductive and respiratory syndrome, wherein the JW-PRRSV vaccine composition is to be administered to a pig.

Administration of the vaccine composition to a pig may be performed by typical vaccine administration methods, and the administration can be performed through enteral or parenteral route, oral, intranasal, intravenous, intramuscular, subcutaneous, endermic or other suitable route, although not limited thereto. Preferably, the composition may be inoculated intramuscularly or intranasally.

The vaccine composition preferably includes 2×10⁵ to 2×10⁷ PFU/ml of a Korean-type porcine reproductive and respiratory syndrome virus.

In addition, the present invention provides a diagnosis kit for a Korean-type porcine reproductive and respiratory syndrome virus including a Korean-type porcine reproductive and respiratory syndrome virus (Accession Number KCTC 12096BP).

The diagnosis kit can be produced according to methods typically used in this technical field.

The diagnosis kit includes tools, reagents, etc. generally used for immunologic analysis in this technical field as well as a Korean-type porcine reproductive and respiratory syndrome virus (Accession Number KCTC 12096BP). These tools/reagents include but are not limited to suitable carriers, markers capable of generating detectable signals, solvents, detergents, buffers, stabilizer, and the like. If the marker is an enzyme, it may include a substrate capable of measuring the enzyme activity and a quenching agent. Suitable carriers include but are not limited to soluble carriers such as physiologically acceptable buffers known in this technical field, such as PBS, insoluble carriers such as polystyrene, polyethylene, polypropylene, polyester, polyacrylonitrile, fluoride resin, crosslinked dextrane, polysaccharide, polymer such as magnetic particles where metal is plated on latex, paper, glass, metal, agarose, and a combination thereof.

In addition, the present invention provides an *in vitro* method for detecting a Korean-type porcine reproductive and respiratory syndrome virus, characterized by detecting a Korean-type porcine reproductive and respiratory syndrome virus (Accession Number KCTC 12096BP) in a cell to be infected or being infected through an antigen-antibody reaction using Korean-type porcine reproductive and respiratory syndrome virus (Accession Number KCTC 12096BP).

The antigen-antibody reaction can be analyzed using tissue immunostaining, radioimmunoassay (RIA), enzyme immunoassay (ELISA), western blotting, immunoprecipitation assay, immunodiffusion assay, complement fixation assay, FACS, protein chip, etc., although not limited thereto.

### Example 1. Isolation of Korean-type Porcine Reproductive and Respiratory Syndrome Virus

### 1.1. Isolation of porcine alveolar macrophages (hereinafter, PAM)

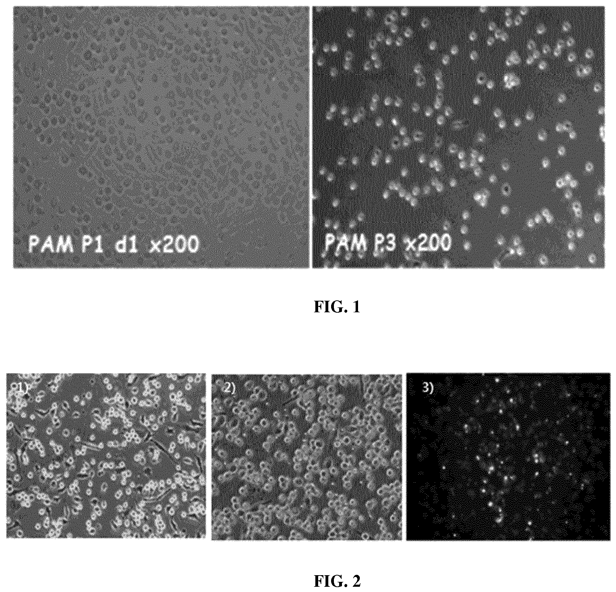

The PAM cells for growing the virus to be isolated were isolated from a germ-free pig being 4 to 8 weeks old (Optifarm-Medipig) which was raised in this company's SPF breeding environment. After anesthetizing the pig, the entire lung tissue including the lung bronchus was carefully separated such that the tissue was not injured. An injection tube was connected inside of the bronchus of the lung, and a prepared phosphate buffer solution (pH 7.2) was injected into the lung tissue through the bronchus of the lung. Upon confirmation of the expansion of the lung tissue, the phosphate buffer solution in the lung tissue was collected by tilting the insertion tube, and the collected phosphate buffer solution was centrifuged to precipitate cells, which were then suspended in a medium containing prepared 10% fetal bovine serum, nonessential amino acid and penicillin/streptomycin. The cell suspension was put into a culture container and cultured at 37°C and the CO₂ concentration of 5%. The primary isolated porcine alveolar macrophages were total 2×10⁸ cells/ml and stored at -80°C. The shape of the cultured porcine alveolar macrophage is shown in Fig. 1.

### 1.2. Isolation of PRRSV and confirmation of PAM infection with PRRSV

The virus used in this experiment was a porcine reproductive and respiratory syndrome virus (hereinafter, PRRSV), which was isolated from an infected pig exhibiting pathological symptoms among JW farm's pigs. Tissues of blood, lung, lymph and tonsil from a pig suspected of being infected were pulverized and suspended in the phosphate buffer solution, and the experiment was conducted to isolate virus from samples that were determined as being virus-positive by the PCR assay.

The PAM isolated in Example 1.1 was seeded into a T25 flask at 2×10⁶ cells/ml. After confirming cell attachment, the cells were inoculated with 10∼100*µ*ℓ of each of the PCR-positive samples such as blood, lung, lymph, and tonsil. Then, when a cytopathic effect (CPE) appeared, the cells were harvested and stored at -80□. T25 flasks containing PAM cells were each inoculated with 100*µ*ℓ of the collected virus, and the virus was confirmed according to the indirect immunofluorescence assay (IFA), and the blind passage culture was continuously performed. More specifically, cells confirmed to be infected with PRRSV were fixed for 5 minutes using 4% paraformaldehyde, and then washed three times for 10 minutes by using a buffer solution. Then, they were treated in a 2% BSA solution for 1 hour in order to prevent non-specific binding of an antibody, and then washed three times for 5 minutes. As a primary antibody, mouse anti-PRRSV Mab 4A5 (JBT Cat#9041) was used, and as a secondary antibody, goat anti-mouse IgG FITC (Santacruz) was used.

The result is shown in Fig. 2.

As shown in Fig. 2, after 96 hours from the inoculation of the isolated PAM with virus, fluorescence could be confirmed in the PAM cells. From this, it was confirmed that the PAM cells w effectively infected with PRRSV.

### 1.3. Isolation of virus clone according to the plaque isolation method

The PAM cells were inoculated in a 6-well plate at a concentration of 3×10⁵cells/well, and after 12 hours, the isolated PRRSV was diluted 10-fold starting from MOI=1 and each of the wells was inoculated with the PRRSV. After culturing for 1 hour, the medium was removed, and the cell layer infected with virus was covered by a DMEM medium in which 0.5% agarose and 10% fetal bovine serum were contained, followed by culturing at 37°C and 5% CO₂. After about 72 hours, the formation of plaques was confirmed with the naked eye, and each of the plaques was independently isolated and purified to isolate a PRRSV clone.

### Example 2. Isolation of JW-PRRSV (KCTC 12096BP) through PRRSV gene analysis

### 2.1. Amplification of PRRSV gene

A phylogenetic analysis was performed from gene information of the ORF5 portion of the isolated PRRSV to classify the virus. Genome RNA of the PRRSV was extracted from 150 1 of PRRSV culture medium using a viral RNA extraction kit (Intron. Korea), following the manufacturer's instructions. RT-PCR was performed in order to synthesize viral cDNA corresponding to the respective gene segments. 2*µ*ℓ of 10pmol ORF5 reverse primer was put into 10*µ*ℓ of the extracted RNA, and heated at 80°C for 3 minutes, and then cooled. 1*µ*ℓ of RNA inhibitor (Promega, U.S.A), 2*µ*ℓ of 5X RT buffer solution (50mM Tris-HCl (Ph 8.3), 75mM KCl, 3mM MgCl₂, 10mM DTT), 10mM dNTP (Promega, U.S.A), and 1*µ*ℓ of M-MLV reverse transcriptase (Promega, U.S.A) were added thereto, followed by amplification at 37°C for 1 hour and 30 minutes. PCR was performed using the synthesized cDNA as a template. PCR was performed by adding sterile distilled water (16*µ*ℓ), cDNA (2*µ*ℓ), and primer (1*µ*ℓ) in a premix tube (Intron), performing 34 cycles of a series of reactions consisting of 94°C for 5 minutes, denaturation at 94°C for 20 seconds, annealing at 60°C for 30 seconds, and extension at 72°C for 45 seconds, and then incubation at 72°C for 5 minutes. For the primers used in the experiment, VR2332 (US strain, Genbank accession number, U87383), whose base sequence was reported for base sequence analysis of ORF5 gene, was used as a reference, and a forward primer (CCA TTC TGT TGG CAA TTT GA) and a reverse primer (CAC CTT TAG GGC ATA TAT CAT) were used.

### 2.2. Phylogenetic analysis of isolated PRRSV

The PCR products were subjected to electrophoresis in 1% agarose gel, and then was isolated using a gel extraction kit (Macherey-Nagel, Germany), and these PCR products were connected to a multicloning site of lac Z gene of a pGEM-T plasmid vector, and each of the genes was subjected to cloning using *E. coli* DH5α competent cell as a host cell. More specifically, *E. coli* DH5α being stored at -70°C was melted in ice. 50-70*µ*ℓ of it were added to a tube in which the connected plasmid DNA was present. After leaving it in ice for 20 minutes, a heat shock was applied at 42°C for 90 seconds, and then it was left in ice for 3 minutes again. 1ml of LB (Luria-Bertani) medium was put into the tube and incubated in an incubator at 37°C for 1 hour. Then, the cells were uniformly seeded in a LB agar medium containing ampicillin (300mg/ml), X-gal, IPTG, etc., and cultured at 37°C for one night. After culturing, white colonies were selected and used for plasmid DNA extraction for base sequence analysis. Each of the selected white colonies was put into a 5ml LB agar plate containing ampicillin, cultured in an incubator at 37°C for one night, and then centrifuged at 3000rpm for 10 minutes. After centrifugation, plasmid DNA was extracted using QIAprep Spin Miniprep kit (Qiagen, U.S.A.). The gene information of the purified plasmid DNA was analyzed with Workbench Version 4 (CLC Bio, Arthus, Denmark) software, and the phylogenetic analysis diagram was prepared. The genetic variation was analyzed by comparing nucleotide sequences of original virus species and the passage cultured sequence.

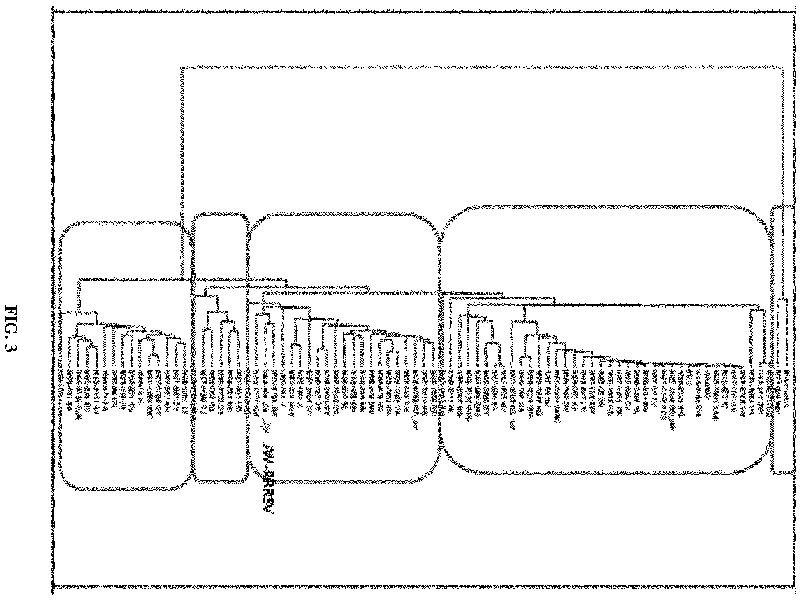

The result is shown in Fig. 3.

As shown in Fig. 3, it was confirmed that the isolated PRRSV is a novel Korean-type PRRSV which is different from North America type PRRSV in 18% of nucleotides. The novel Korean-type PRRSV was named as JW-PRRSV, and deposited with accession number KCTC 12096BP on December 2, 2011.

### Example 3. Confirmation of immunogenicity of a vaccine developed using the Korean-type PRRSV (hereinafter, JW-PRRSV) (passage 5, passage 91)

### 3.1. Clinical measurements after inoculation with JW-PRRSV

In order to develop the newly isolated JW-PRRSV as a vaccine, pigs were inoculated with JW-PRRSVs of passage 5 and passage 91, and then the effect was observed. More specifically, 12 colostrum-derived pigs that were PRRSV-negative and were SPF (specific-pathogen-free) were grouped into total four groups: Passage 5 JW-PRRSV, Passage 91 JW-PRRSV, MLV vaccine (commercial vaccine), and negative control, to confirm immunogenicity (Table 1).

**Table 1**

| CD-pig Subject Number | Contents |
|---|---|
| B09-0 11 | Group inoculated with 2ml of Passage 5 JW-PRRSV |
| B09-012 | |
| B09-013 | |
| H10-002 | Group inoculated with 2ml of Passage 91 JW-PRRSV |
| H10-004 | |
| B09-014 | |
| B09-008 | Group inoculated with MLV vaccine |
| B09-009 | |
| B09-010 | |
| H10-005 | Negative control |
| H10-006 | |
| H10-007 | |

Each group was inoculated with 2ml of virus at 1×10⁵ PFU/ml, and after 1 day, 3 days, 6 days, 10 days, 15 days, 21 days, 28 days and 35 days from the inoculation, clinical symptoms such as body temperature/motility/food intake, etc. were observed. After 37 days from the inoculation, the pigs were challenged with original virulent JW field-virus at 1×10⁴ PFU/ml and monitored for the same items. More specifically, body temperature and motility were measured at the same time twice a day during the experimental period, and the motility was monitored in such a manner that scores were given from 0 score to 4 scores at an interval of 0.5 score through the observation of the subjects (0 score: no motility, 1 score: weak motility, 2 scores: normal motility, 3 scores: good motility, 4 scores: very good motility). In addition, for food intake, restricted feeding was performed depending on the weight. When all food was eaten within 10 minutes, the food intake was determined as very good (4 scores). Scores were given from 0 score to 4 scores at an interval of 0.5 score depending on the time taken to consume all the food. The overall motility and the food intake were evaluated by the sum of the scores obtained as above.

The result is shown in Table 2.

**Table 2**

| Experimental group | Motility | Food intake |
|---|---|---|
| Group inoculated with Passage 5 JW-PRRSV | The group was managed with good food intake and motility until the experiment was terminated. | |
| Group inoculated with Passage 91 JW-PRRSV | The group was managed with good food intake and motility until the experiment was terminated. | |
| Group inoculated with MLV vaccine | The group was managed with good food intake and motility until the experiment was terminated. | |
| Control | The day of challenge: After 10 to 15 minutes from inoculation, the subject H10-005 vomited and showed reduced food intake. The subject H10-006 showed reduced motility (when feeding after inoculation, the pig was not willing to take the food, but the pig took all of the food) | |
| | 1 day after challenge: Good food intake and motility. After 15 hours from inoculation, the body temperature increased by 1.6 degree as compared to the date of challenge. After 24 hours from inoculation, the body temperature increased by 0.96 degree. | |
| | 2 days after challenge: Good food intake and motility. | |

As shown in Table 2, analysis of the scores shows that the Groups inoculated with Passage 5 JW-PRRSV, Passage 91 JW-PRRSV and MLV vaccine showed good food intake and motility until the experiment was terminated. Therefore, it was confirmed that both Passage 5 JW-PRRSV and Passage 91 JW-PRRSV lead to the virus protection equivalent to the commercial MLV vaccine.

### 3.2. Measurement of the antibody titer after inoculation with Korean-type PRRSV (JW-PRRSV)

In order to evaluate the vaccine effect of JW-PRRSV, virus was inoculated under the same conditions as in Example 3.1 and then the antibody titer was measured. After 37 days from inoculation, the pigs were challenged with original virulent JW field-virus at 1×10⁴ PFU/ml and monitored for the same items as in Example 3.1. After 14 days from challenge, an autopsy was conducted on all subjects, and quantitative PCR was performed for lung, lymph, tonsil, lien, liver, brain, and kidney. In order to enable a vaccine virus to lead to the protection against a field-virus, primarily, a serum test should be positive for antibody formation, and there should be virus neutralizing ability. To check whether the antibody titer in the inoculated animal became positive, the ELISA (HerdChek: PRRS 2XR ELISA kit (IDEXX laboratories, Westbrook, ME, USA)) method was used. The presence or absence of PRRSV antibody was expressed with S/P ratio, and when the S/P ratio was 0.4 or higher, it was determined as being positive.

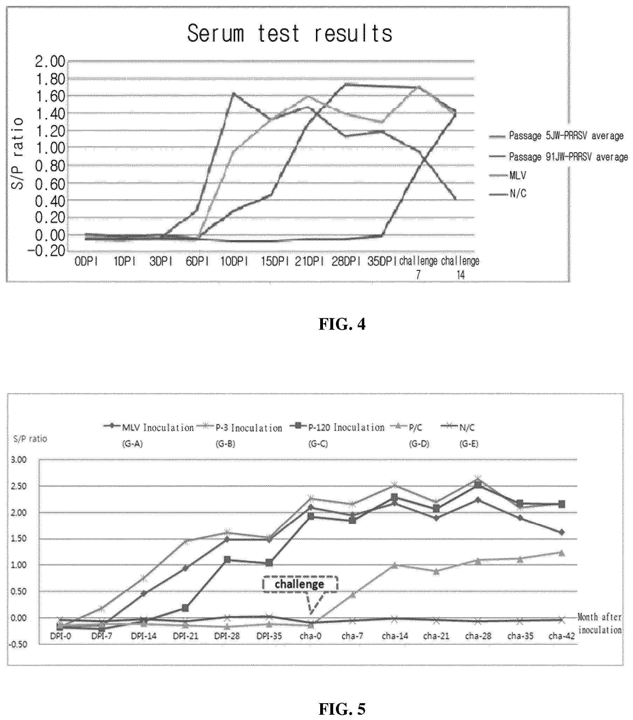

The result is shown in Table 3 and Fig. 4.

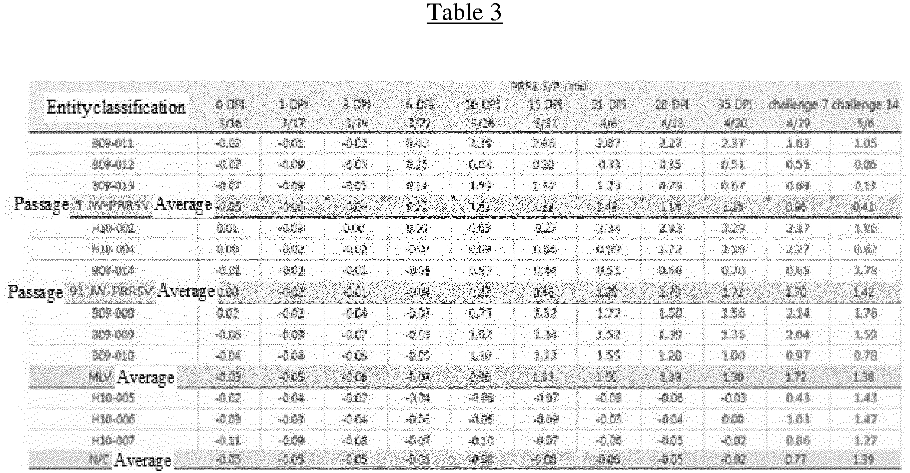

As shown in Table 3 and Fig. 4, it could be confirmed that after 10 days from the inoculation, in the experimental groups inoculated with Passage 5 JW-PRRSV, Passage 91 JW-PRRSV and MLV vaccine, except for control, an increase in antibody titer was observed, and on 35 days after inoculation, in all experimental groups, the antibody titer became positive.

### 3.3. Measurement of virus titers by JW-PRRSV

In order to evaluate the vaccine effect of JW-PRRSV, the groups were inoculated with virus under the same conditions as in Example 3.1, and then PRRSV virus titers in blood and tissues after autopsy were measured. After 37 days from inoculation, the pigs were challenged with original virulent JW field-virus at 1×10⁴ PFU/ml and monitored for the same items as in Example 3.1.

The result is shown in Table 4.

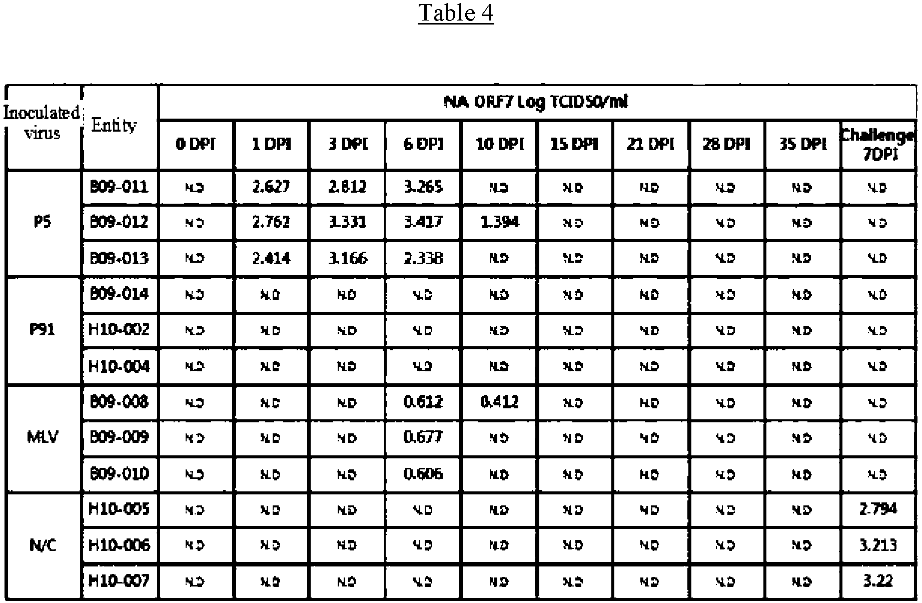

As shown in Table 4, in the group inoculated with Passage 5 JW-PRRSV, there was one subject in which virus was detected in blood until 10 days from the inoculation. In the group inoculated with Passage 91 JW-PRRSV, no virus was detected in blood after the inoculation and even after challenge. In the group inoculated with commercial live vaccine MLV, some virus was detected on 6 days after the inoculation, and no virus was detected after challenge. In the negative control, a considerable amount of virus was detected in blood on 7 days after challenge.

### Example 4 Confirmation of safety and immunogenicity of a vaccine developed using Korean-type PRRSV (JW-PRRSV)

20 colostrum-derived pigs that showed PRRSV-negative and were SPF (specific-pathogen-free) were grouped into total five groups: MLV vaccine (commercial vaccine; G-A), Passage 3 JW-PRRSV (G-B), Passage 120 JW-PRRSV (G-C), negative control 1 (negative control against challenge; G-D), and negative control 2 (negative control in the entire experiment; G-E). Each group was inoculated with 2 ml of virus at 1×10⁵ PFU/ml, and after 7 days, 14 days, 21 days, 28 days and 35 days after inoculation, clinical symptoms such as body temperature/motility/food intake, etc. were observed, and the measurement of virus titers in blood and antibody titers, blood cell analysis, and clinical symptom analysis were conducted by gathering blood. And then, after 42 days from inoculation, the pigs were challenged with original virulent JW field-virus at 1×10⁴ PFU/ml and monitored for the same items. In order to enable a vaccine virus to lead to the protection against a field-virus, primarily, a serum test should be positive for antibodies, and there should be virus neutralizing ability. To check whether the antibody titer in the inoculated animal became positive, the ELISA (HerdChek: PRRS 2XR ELISA kit (IDEXX laboratories, Westbrook, ME, USA)) method was used. To check whether the neutralizing antibody titers were induced, the VN (virus neutralization) test was used.

### 4.1. Measurement of virus titers according to the RT-PCR method

In order to measure the amount of PRRSV, a primer (ORF7) was manufactured with reference to VR2332 (US strain, Genebank accession number, U87382) whose base sequence was already reported (forward primer: ATG ATG RGC TGG CAT TACT, reverse primer: ACA CGG TCG CCC TAA TTG). Genome RNA of the PRRSV was extracted from PRRSV culture medium (150*µ*ℓ) using a viral RNA extraction kit (Intron. Korea), following the kit manufacturer's instructions, and RT-PCR was performed in order to synthesize viral cDNA corresponding to the respective gene segments. 2*µ*ℓ of 10pmol ORF5 reverse primer was put into 10*µ*ℓ of the extracted RNA, and heated at 0°C for 3 minutes, and then cooled. 1*µ*ℓ of RNA inhibitor (Promega, U.S.A), 2*µ*ℓ of 5X RT buffer solution (50mM Tris-HCl (Ph 8.3), 75mM KCL, 3mM MgCl₂, 10mM DTT), 10mM dNTP (Promega, U.S.A), and 1*µ*ℓ of M-MLV reverse transcriptase (Promega, U.S.A) were added thereto, followed by amplification at 37°C for 1 hour and 30 minutes. PCR was performed under the following conditions using the synthesized cDNA (2*µ*ℓ), Sybr green dye 10ul (Bio-Rad Korea), sterile distilled water (6*µ*ℓ), and each primer (1*µ*ℓ): 40 cycles of a series of reactions consisting of 95°C for 3 minutes, denaturation at 95°C for 20 seconds, annealing at 60°C for 20 seconds, and extension at 72°C for 30 seconds, followed by 71 cycles of 55°C for 1 minute and 60°C for 10 seconds. Since RT-PCR uses known amounts of serially diluted DNA as standard examples, a calibration curve was prepared by plotting the number of cycles (threshold cycle (Ct value)) at which a certain amount of amplified product is obtained in an exponential amplification phase on the horizontal axis and the initial DNA amount on the vertical axis. For samples of unknown concentrations, the reaction was performed under the same conditions to obtain the Ct values and thereby determine the target DNA amounts.

The result is shown in Table 5.

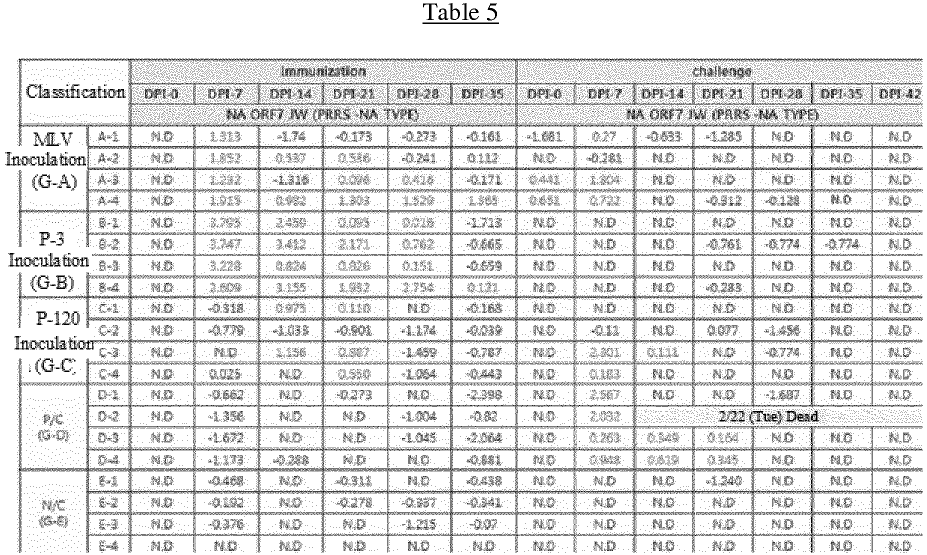

As shown in Table 5, in the group inoculated with MLV vaccine (G-A), after 7 days from inoculation, in all subjects, virus was detected, and there was a subject in which virus was detected until 35 days. In the group inoculated with Passage 5 JW-PRRV (G-B), after 7 days from inoculation, in all subjects, virus having a high titer was detected, and virus was continuously detected until 28 days, and there was a subject in which virus was detected until 35 days. In the group inoculated with Passage 120 JW-PRRV (G-C), after 14 days from inoculation, in two subjects, virus was detected, and virus remained until 21 days after inoculation, but no virus was detected from 28 days. From this result, it can be known that this vaccine showed virus titers 6 times lower than commercial MLV vaccine (G-A) and the permanence in blood was also short. In addition, in the group inoculated with MLV vaccine (G-A), in all subjects, virus was detected, but in the group inoculated with Passage 120 JW-PRRV (G-C), virus was detected only in two subjects. After 42 days from challenge, in the group inoculated with MLV vaccine (G-A), after 7 days from the inoculation, virus was detected in the blood of three subjects, and in the group inoculated with Passage 5 JW-PRRV (G-B), in all four subjects, no virus was detected. This is deemed to be homologous challenge protection (Report: Colloquium on Prospects for Development of an Effective PRRS Virus Vaccine, August 13, 2007 - D.L. Rock, PhD, University of Illinois). In the group inoculated with Passage 120 JW-PRRV (G-C), after 7 days from challenge, in two subjects, virus was detected. In the negative group (G-D), after 7 days from challenge, in two subjects, a considerable amount of virus was detected in blood, and out of these subjects, one died after 14 days from the inoculation. An autopsy was conducted on this subject, and the virus titers for lung, lymph, tonsil, kidney, bronchus, liver, etc. were measured.

The result is shown in Table 6.

**Table 6**

| Measurement of virus titers in tissues for D-2 of G-D | |
|---|---|
| Autopsy Blood | Negative (Log TCID₅₀/ml) |
| lung | 2.767 |
| liver | -0.171 |
| kidney | -0.366 |
| bronchus | 0.681 |
| lymph | 0.107 |
| tonsil | 2.685 |

As shown in Table 6, it could be confirmed that in the lung and tonsil of the negative control who died, a considerable amount of virus was detected.

### 4.2. Measurement result of antibody titers in blood

The blood samples obtained from each of the inoculated groups were analyzed by the ELISA (HerdChek: PRRS 2XR ELISA kit (IDEXX laboratories, Westbrook, ME, USA) method to measure the antibody titers in blood against PRRSV. The presence or absence of the formation of the antibody against PRRSV was expressed with S/P ratio, and when the S/P ratio was 0.4 or higher, it was determined as positive.

The result is shown in Table 7 and Fig. 5.

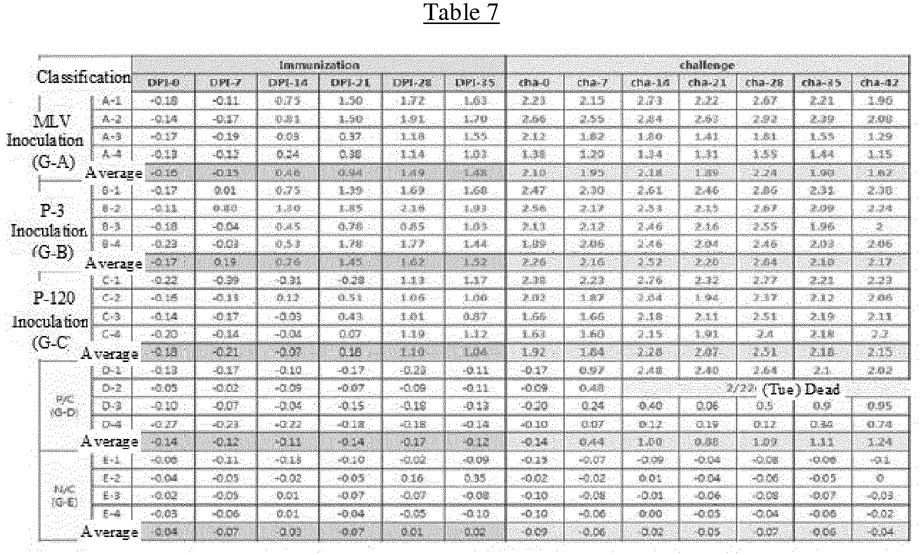

As shown in Table 7 and Fig. 5, in the group inoculated with commercial MLV vaccine (G-A), after 14 days from inoculation, in two subjects, virus titers became positive, and after 25 days, all of the four subjects became positive for virus. In the group inoculated with Passage 5 JW-PRRV (G-B), after 7 days from inoculation, in one subject, virus titers became positive, and after 14 days, all of the four subjects became positive for virus. In the group inoculated with Passage 120 JW-PRRV (G-C), after 21 days from inoculation, two subjects became positive, and after 14 days, all of the four subjects became positive for virus. The results shows that the group inoculated with Passage 120 JW-PRRV (G-C) is a candidate substance which can sufficiently induce antibody titers while being safe.

### 4.3. Blood cell analysis result

The levels of various blood cells present in blood were measured in the blood samples collected from each of the inoculated groups using an automatic blood cell counter.

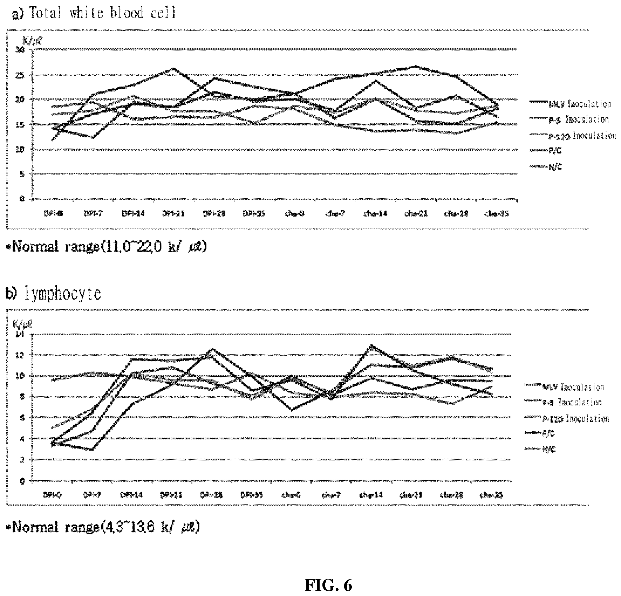

The result is shown in Fig. 6.

As shown in Fig. 6, it could be confirmed that in the group inoculated with Passage 3 JW-PRRV (G-B), white blood cells slightly increased, but in other groups, the change in white blood cell number was not significant until the experiment was terminated (a). In addition, the measurement result of lymphocytes in blood showed that even after inoculation of the vaccine candidate group, the lymphocyte number remained normal at 4.3 to 13,600cells/*µ*ℓ (b). This result shows that the vaccine candidate substance of the present invention is a safe substance that does not lead to inflammation in a subject.

### 4.4. Clinical analysis result

After challenge with a vaccine, clinical symptoms appearing on pigs were observed. Body temperature and motility were measured at the same time twice a day during the experimental period. For the motility, scores were given from 0 point to 4 scores at an interval of 0.5 score through individual observation (0 score: no motility, 1 score: weak motility, 2 scores: normal motility, 3 scores: good motility, 4 scores: very good motility). For food intake, feeding was restricted depending on the weight. When all food was consumed within 10 minutes, the food intake was determined as very good (4 scores). Scores were given from 0 score to 4 scores at an interval of 0.5 score, according to the time taken to consume all the food.

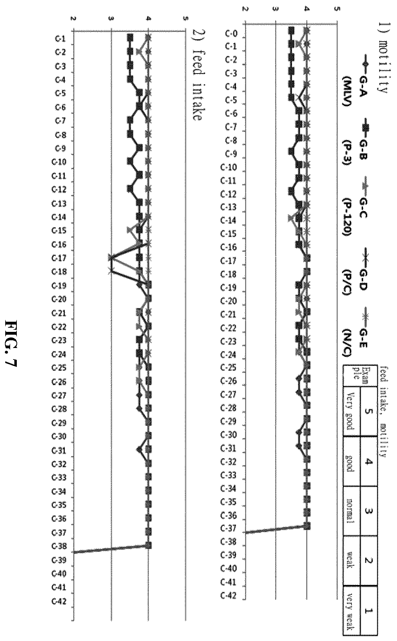

The result is shown in Table 8 and Fig. 7.

**Table 8**

| Classification | 2/08 (Tue)∼2/15 (Tue) DPI-Q-7 | 2/16 (Wed)∼2/22(Tue ) | 2/23 (Wed)∼3/1(Tue) | 3/2 (Wed)∼ |
|---|---|---|---|---|
| G-A (MLV) | Jinwang (08-296) P3 challenge: After intramuscular and nasal inoculation with 2ml per dose, no symptoms such as reduced food intake and motility were found. | - Good food intake and motility | - Good food intake and motility | - Good food intake and motility |
| | | | - A-1, which was isolated due to blue ear symptom in the left ear, was improved in food intake from normal to good on 16 days. | - A-1, isolated from the group and separately managed, shows good food intake and motility. |
| G-B (P-3) | - The food intake and motility after challenge have no difference during from immunization. | - Good food intake and motility | - Good food intake and motility | - Good food intake and motility |
| | - Weak mobility due to abnormalities in the front and rear leg joints, normal food intake | - After 16 days from challenge, B-4 gradually recovered good food intake and motility. | - On 16 days after challenge, B-4 recovered normal food intake and motility. | |
| G-C (P-120) | - 1 day: C-3 showed slightly reduced food intake and motility than other pigs in the group. | - Good food intake and motility | - Good food intake and motility | - Good food intake and motility |
| | | - C-3 showed reduced food intake on 12 days after change. As of 14 days, it shows a slightly slower food intake speed than the other subjects of the group although it is recovering. | - C-3 showed good motility and food intake but has a slower food intake speed than the others in the group. | |
| | - 2 days: recovery to normal food intake and motility | | | |
| G-D (P/C) | - 6 days: D-2 showed slightly reduced food intake and motility than the other pigs in the group. | - 10 days: D2 exhibited red spots in the whole body (similar to exudative epidermitis symptoms) | - Good food intake and motility | - Good food intake and motility |
| | - 7 days: recovery to normal food intake and motility | -13 days: The condition of D-2 suddenly got worse during the afternoon management | | |
| | | - 14 days: D-2 died (refer to autopsy result and inspection result materials) | | |
| G-E (N/C) | - Good food intake and motility | - Good food intake and motility | - Good food intake and motility | - Good food intake and motility |

As shown in Table 8 and Fig. 7, although the group inoculated with Passage 3 JW-PRRSV (G-B) showed somewhat lower motility and food intake overall, it showed overall good food intake and motility with nothing unusual.

### Example 5. Preparation of JW-PRRSV vaccine

### 5.1. Passage method and preservation

MARC-145 cells were inoculated with PRRS original virulent virus (120 passages) of more than 10^{4.0}TCID₅₀/ml which was confirmed in the identification test and the immunogenicity test. The cells were cultured at 37°C for 4-5 days to obtain virus, which was then freeze-dried or frozen and stored at -80°C. Virulent PRRSV was obtained in the same manner as PRRS original virulent virus, and then freeze-dried or frozen and stored at -80°C. The original virulent virus was not cultured for more than 3 passages, and the virus titer was at least 10^{6.0}TCID₅₀/ml.

### 5.2. Bulk production and virus content test

MARC-145 or PAM cells were cultured using 850cm² roller bottle, in a medium for cell proliferation, and passaged every 3 to 5 days. When a single layer of the 850cm² roller bottle cultured-cells was formed, the medium for cell proliferation was removed, and the cells were inoculated with the target virus in an amount sufficient to cover the cells, and then adsorbed at 37°C for 1 hour. Then, the inoculation liquid was removed and a medium for cell proliferation was added, followed by roller bottle culture at 37°C for 4-5 days. The centrifuged virus culture medium was subjected to decimal serial dilution, inoculated in a 96-well plate where MARC-145 or PAM cells were cultured, and then cultured at 37°C for 7 days while observing the cytopathic effect (CPE). When 80-90% of CPE was expressed after virus culturing, it was aseptically collected and frozen and stored at -80°C.

### 5.3. Preparation of test vaccine

* A test vaccine was prepared by using isolated virus as an antigen and a protective agent. Three types of test vaccines were prepared by applying a sterile phosphate buffer solution and using a freeze dryer (production number: 60 PRRS 01, 60 PRRS 02, 60 PRRS 03). The contents of the prepared test vaccines are shown in Table 9, Table 10 and Table 11.

**Table 9**

| 60 PRRS 01 | | |
|---|---|---|
| Raw drug | Content | Bulk amount |
| PRRS | 10^{6.5}TCID₅₀/ml | 600ml |
| TPGG | | 150ml |
| Total | | 750ml |

**Table 10**

| 60 PRRS 02 | | |
|---|---|---|
| Raw drug | Content | Bulk amount |
| PRRS | 10^{6.6}TCID₅₀/ml | 620ml |
| TPGG | | 156ml |
| Total | | 776ml |

**Table 11**

| 60 PRRS 03 | | |
|---|---|---|
| Raw drug | Content | Bulk amount |
| PRRS | 10^{6.4} TCID₅₀/ml | 640ml |
| TPGG | | 160ml |
| Total | | 800ml |

As TPGG (trehalose), instead of the conventionally used LPGG (lactose), was used as a protective agent, contamination which may be generated when preparing a live vaccine due to existing protective agents could be minimized and the stability of the vaccine was increased. The comparison table for the virus content for protective agents is shown in Table 12.

**Table 12**

| Raw drug | Virus Content | Content after freeze drying |
|---|---|---|
| PRRS | 10^{6.5}TCID₅₀/ml | |
| TPGG | | 10^{5.8}TCID₅₀/ml |
| Total | | 10^{5.8}TCID₅₀/ml |

### 5.4. Stability test of test vaccines

In order to test the stability of the test vaccines prepared in Example 5.3, the following were assessed at the time of preparation, after 3 months from the preparation, and after 6 months from the preparation: characteristics, vacuum test, hydrogen-ion concentration, moisture content, sterility test, mycoplasma test, adventitious virus test, content test, and titer test.

The result of the safety test is shown in Table 13.

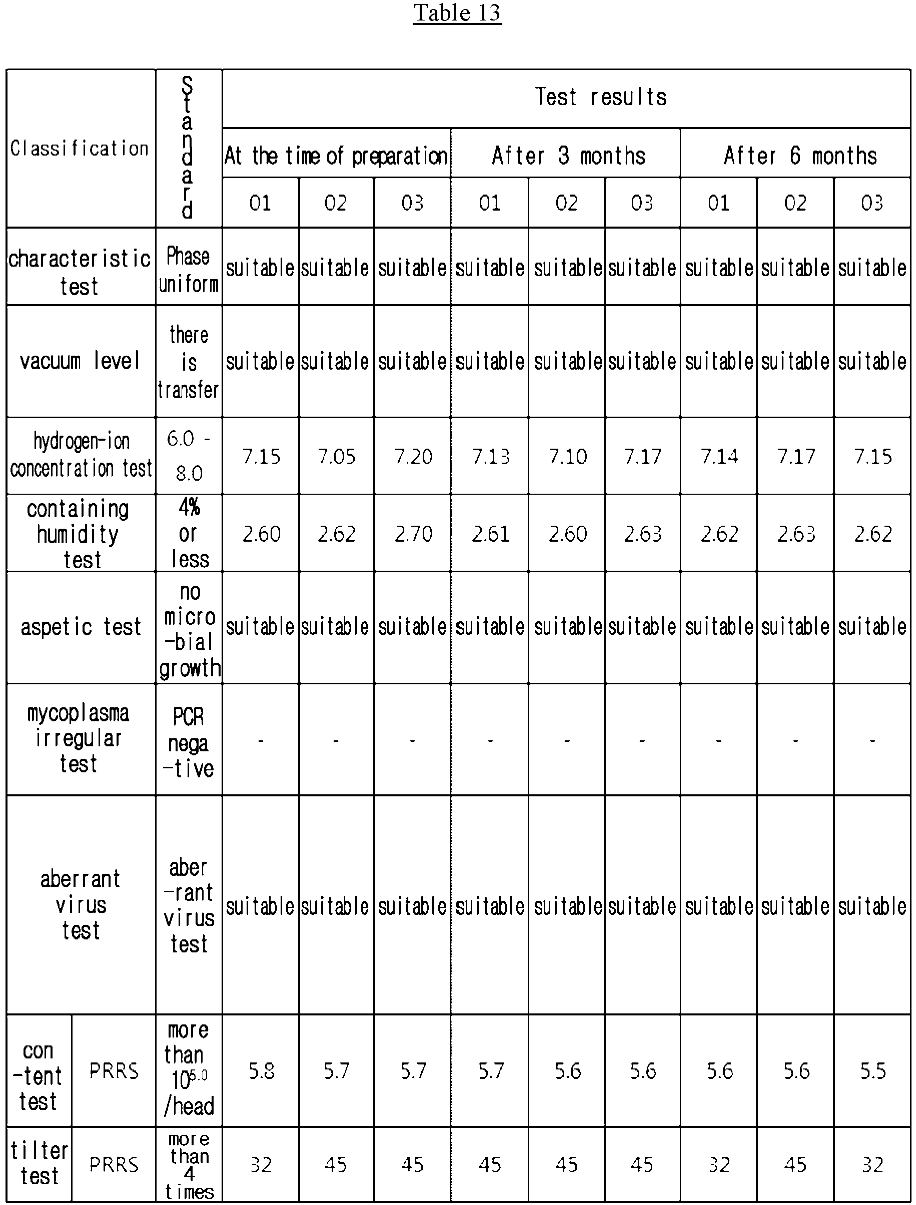

As shown in table 13, it was confirmed that the prepared test vaccines 60 PRRS 01, 60 PRRS 02 and 60PRRS 03 are suitable in all of the stability test items at the time of preparation, after 3 months from the preparation, and after 6 months from the preparation. Therefore, it can be understood that the vaccine using JW-PRRSV, which is the novel Korean-type PRRSV of the present invention, is a suitable vaccine in terms of stability as well.

### Example 6. Confirmation of safety of test vaccines

In order to confirm safety, which is the most important in a live vaccine, test animals, mouse and guinea pig, and the target animal, pig, were inoculated with the test vaccines, and the survival rates for 7 days or 21 days were observed. As the inoculated test vaccines, 60 PRRS 01, 60 PRRS 02 and 60 PRRS 03 prepared in Example 5.3 were used, The test animals and the target animal were inoculated with the test vaccines at the time of preparation, after 3 months from the preparation, and after 6 months from the preparation, and the survival rate was observed.

### 6.1. Confirmation of safety of test vaccines in mouse

8 mice weighing 15-20 g were intraperitoneally inoculated with 0.5 ml of each of the test vaccines (at the time of preparation, 3 months after the preparation, 6 months after the preparation) and observed for 7 days.

The result is shown in Table 14.

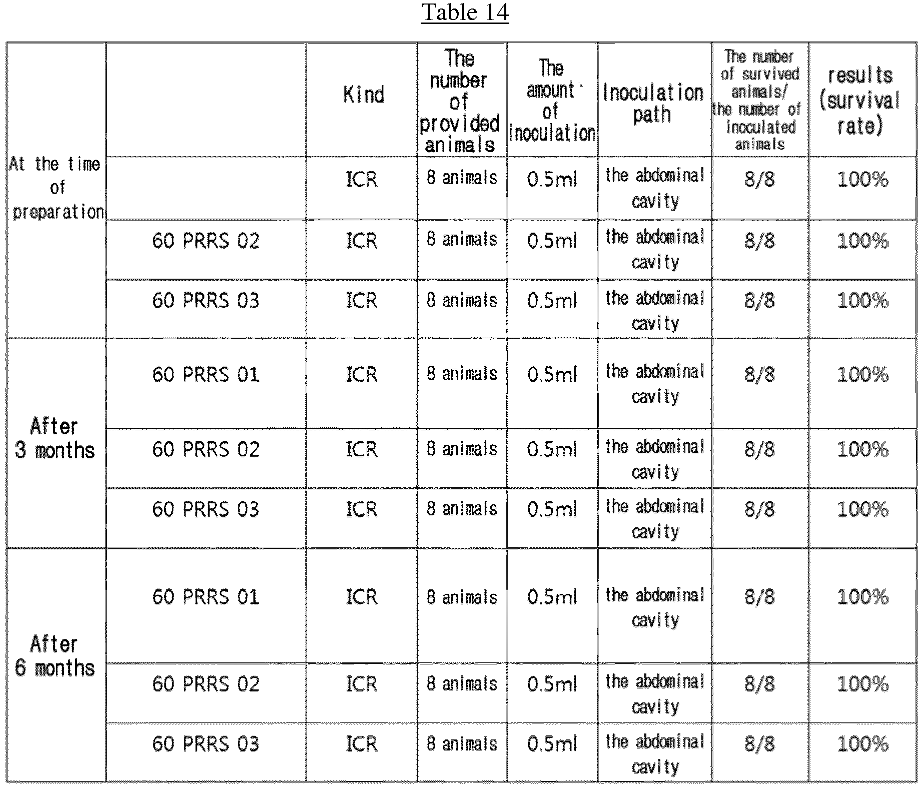

As shown in Table 14, all subjects inoculated with the test vaccines survived, and thus it was confirmed that these may be safe vaccine candidates.

### 6.2. Confirmation of safety of test vaccine in guinea pig

4 guinea pigs weighing 300-350g were used. Two of them were inoculated with two doses of the test vaccines in muscle or subcutaneously, the others were inoculated with two doses of the test vaccines into the abdominal cavity (at the time of preparation, after 3 months from the preparation, after 6 months from the preparation), and they were observed for 7 days.

The result is shown in Table 15.

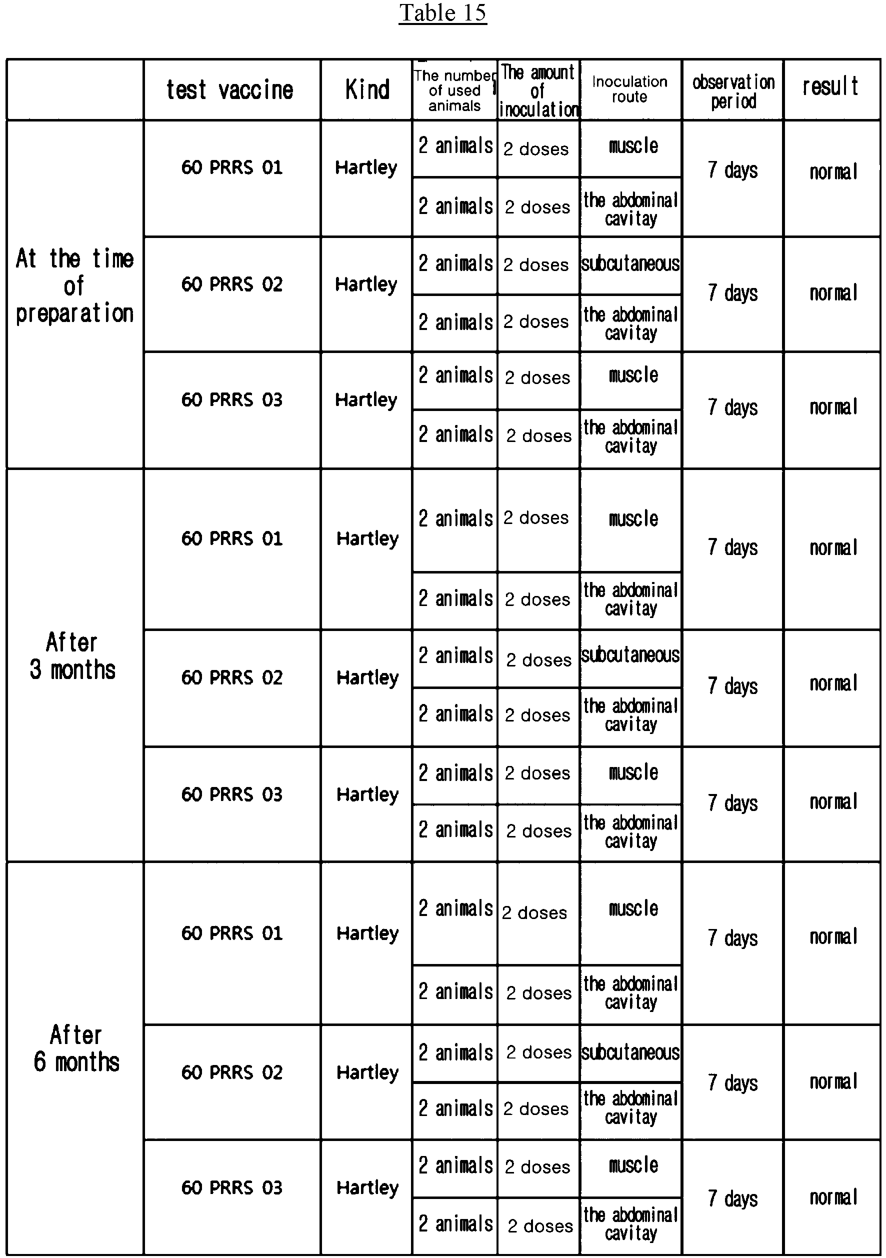

As shown in Table 15, subjects in all of the groups inoculated with the test vaccines survived without any abnormality for 7 days, and thus it was confirmed that these may be safe vaccine candidates.

### 6.3. Confirmation of safety of test vaccines in piglet

In the target animal, piglet, the safety of the test vaccines (at the time of preparation, after 3 months from the preparation, after 6 months from the preparation) was confirmed. 18 healthy pigs (4-6 weeks) showing negative to PRRSV antibody and weighing 8-10 kg were inoculated with 10 doses of each of the vaccines, and then observed for 21 days.

The result is shown in Table 16.

**Table 16**

| test vaccine | | The number of used animals | The amount of inoculation | Inoculation route | observation period | result |
|---|---|---|---|---|---|---|
| At the time of preparation | 60 PRRS 01 | 2 animals | 10 doses | muscle | 2 animals | normal |
| | 60 PRRS 02 | 2 animals | 10 doses | muscle | 2 animals | normal |
| | 60 PRRS 03 | 2 animals | 10 doses | muscle | 2 animals | normal |
| After 3 months | 60 PRRS 01 | 2 animals | 10 doses | muscle | 2 animals | normal |
| | 60 PRRS 02 | 2 animals | 10 doses | muscle | 2 animals | normal |
| | 60 PRRS 03 | 2 animals | 10 doses | muscle | 2 animals | normal |
| After 6 months | 60 PRRS 01 | 2 animals | 10 doses | muscle | 2 animals | normal |
| | 60 PRRS 02 | 2 animals | 10 doses | muscle | 2 animals | normal |
| | 60 PRRS 03 | 2 animals | 10 doses | muscle | 2 animals | normal |

As shown in Table 16, in piglets, no hypersensitivity reaction was observed within 1-2 hours after inoculation, and it was confirmed that during the observation for 21 days, they all survived without side effects such as suppuration in an inoculated site, necrosis, fever, and diarrhea. Therefore, it can be understood that the test vaccines of the present invention have the safety suitable as a vaccine of porcine PRRSV.

### Example 7. Confirmation of safety of test vaccines in SPF piglet

The degree of virus infection in blood and tissue when the target animal was inoculated with each of the test vaccines prepared in Example 5 at a high concentration was assessed. Also, it was assessed whether the pathogenicity of virus reverted to its original wild-type pathogenicity or higher pathogenicity when the target animal was inoculated with serial passage virus. Starting from the primary inoculation of two SPF pigs, pigs were inoculated with 2ml of attenuated 120 passage JW-PRRSV 2X10⁶ PFU/ml intramuscularly and intranasally. The degree of virus infection in blood after 1 day, 3 days and 7 days and in the lung, tonsil and lymph after an autopsy was assessed. The age and weight, virus history and test schedule of the inoculated subjects are shown in Table 17, and the test result of the degree of virus infection according to the test schedule is shown in Table 18.

**Table 17**

| Classification | Subject | Age (day) | Weight (kg) | Inoculated virus | Test date |
|---|---|---|---|---|---|
| Primary | 100-2 | 145 | 23.4 | P120 6.3 TCID₅₀/ml | 2010. 9.1 ∼ 9.7 |
| | 100-4 | | 26.6 | P120 6.3 TCID₅₀/ml | |
| secondary | 100-1 | 164 | 18.7 | Specimen from primary inoculated subject | 2010. 9.20 ∼ 9.27 |
| | 100-5 | | 17.3 | " | |
| tertiary | H10-009 | 212 | 34.1 | Specimen from secondary inoculated subject | 2010. 10.13 ∼ 10.21 |
| | H10-011 | | 38.2 | " | |
| quaternary | H10-013 | 227 | 36.5 | Specimen from teritary inoculated subject | 2010. 10.27 ∼ 11.03 |
| | H10-014 | | 26.1 | " | |
| quinary | 104-3 | 127 | 19.8 | Specimen from quaternary inoculated subject | 2010. 11.25 ∼ 12.01 |
| | 104-4 | | 16.7 | " | |

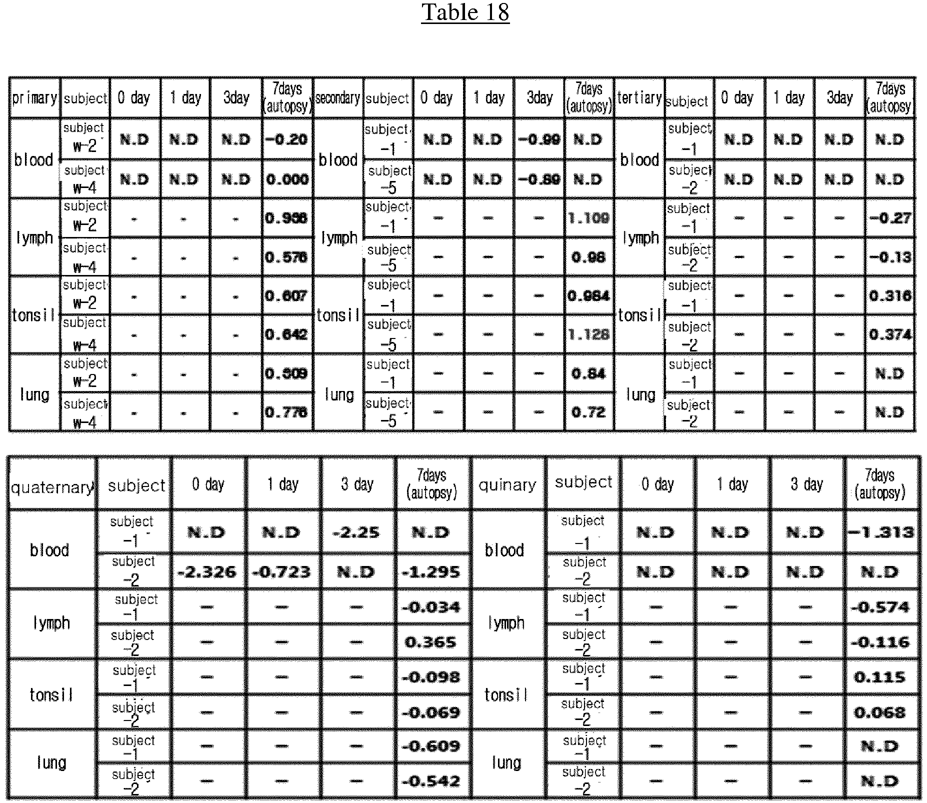

As shown in Table 18, the measurement results of virus titers in the blood and tissue after the primary to quinary passages in the target animals showed that no virus was detected in the blood until the quinary inoculation. In the tissue, low virus titer was observed, and during the 5 passages, the amplification or transfer of virus and clinical symptoms were not observed (*N.D: nondetection). In addition, the results of observations from the time of inoculation to autopsy showed that they all survived without side effect such as suppuration in an inoculated site, necrosis, fever, and diarrhea.

### Example 8. Confirmation of virus shedding

Generally, upon first inoculation with a live vaccine, viral shedding of the vaccine virus can be observed for 3 to 5 weeks. Since a vaccine virus results in no abnormal symptoms, side effects of viral shedding of the live virulent vaccine virus may be a problem. The viral shedding time of the vaccine candidates of the present invention was assessed and compared with existing commercial vaccine. The experimental groups were classified into the group inoculated with MLV vaccine (A), the group inoculated with 5 Passage JW-PRRSV (B), and the group inoculated with 120 Passage JW-PRRSV (C). Nasal samples were obtained from the subjects of each group after 7 days, 12 days, 15 days, 19 days, 22 days, 26 days, 29 days, 33 days, and 36 days from the inoculation, and after 0 day, 7 days, 14 days, 21 days, 28 days, 35 days and 42 days after challenge. Viral shedding for each group was assessed by conducting PCR using virus specific primers. For the primer sequences, the sequences shown in Table 19 were used. PCR was performed in the same manner as the existing method, and 1% agarose gel was used for identification.

**Table 19**

| Primer name | Sequence (5' → 3') | SEQ ID NO : |
|---|---|---|
| Common EU&NA J230_F | atggccagcc agtcaatca | SEQ ID NO : 1 |
| Common EU&NA J068_R | tcgccctaat tgaataggtg a | SEQ ID NO : 2 |

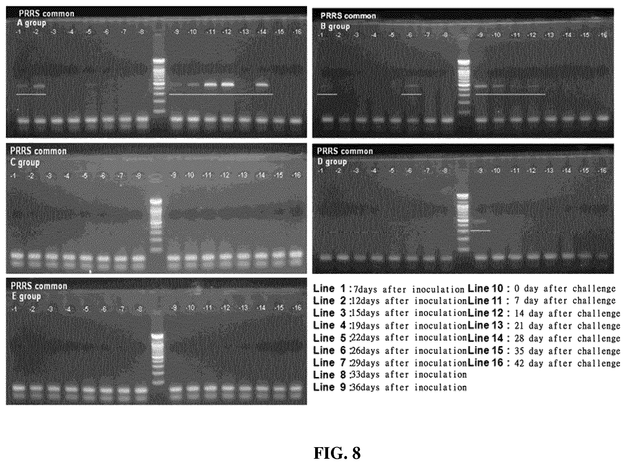

The result is shown in Fig. 8.

As shown in Fig. 8, it was confirmed that in the group inoculated with MLV vaccine (G-A), viral shedding was detected until 12 days from the inoculation with the vaccine, and viral shedding was detected until 28 days from the challenge. In contrast, in the group inoculated with 120 Passage JW-PRRSV (G-C), viral shedding was shown neither during the inoculation period nor after infection with field-virus. This means that this vaccine displays fast viral shedding as compared to the existing commercial vaccine against PRRSV. If long-term viral shedding occurs, the virus will go through several replication rounds, and in this process, its virulence may be reacquired by recombination, so a new virus may appear that has reverted to a highly virulent strain again, resulting in a pathogenic strain. Therefore, the vaccine of the present invention is expected to improve the problem of existing PRRSV vaccines by fast virus shedding.

## Claims

1. A Korean-type porcine reproductive and respiratory syndrome virus (PRRSV) (accession number KCTC 12096BP).

2. A vaccine composition comprising a Korean-type porcine reproductive and respiratory syndrome virus (accession number KCTC 12096BP) as an effective ingredient.

3. The vaccine composition according to claim **2**, wherein the virus is obtained by culturing the virus for 3 to 120 passages.

4. The vaccine composition according to claim **2** or **3**, wherein the vaccine composition further comprises adjuvants, excipients or carriers.

5. The vaccine composition according to any one of claims **2** to **4**, wherein the vaccine composition further comprises a protective agent.

6. The vaccine composition according to claim **5**, wherein the protective agent is trehalose.

7. A Korean-type porcine reproductive and respiratory syndrome virus (PRRSV) (accession number KCTC 12096BP) for use in preventing porcine reproductive and respiratory syndrome.

8. The PRRSV for use according to claim **7**, wherein the PRRSV is to be inoculated intramuscularly or intranasally.

9. The PRRSV for use according to claim **7** or **8**, wherein the PRRSV is to be inoculated at a dose from 2x10⁵ to 2x10⁷ PFU/mL.

10. A diagnosis kit of a Korean-type porcine reproductive and respiratory syndrome virus comprising Korean-type porcine reproductive and respiratory syndrome virus (accession number KCTC 12096BP).

11. An *in vitro* method for detecting Korean-type porcine reproductive and respiratory syndrome virus, **characterized by** detecting a Korean-type porcine reproductive and respiratory syndrome virus (accession number KCTC 12096BP) in a cell to be infected or being infected through an antigen-antibody reaction using Korean-type porcine reproductive and respiratory syndrome virus (accession number KCTC 12096BP).

12. The *in vitro* method according to claim **11**, wherein the antigen-antibody reaction analysis is performed using at least one method selected from a group consisting of tissue immunity staining, radio immunoassay (RIA), enzyme immunoassay method (ELISA), Western Blotting, Immunoprecipitation Assay, Immunodiffusion Assay, Complement Fixation Assay, FACS, and protein chip.

## Patentansprüche

1. Virus des koreanischen Reproduktions- und Atemwegssyndrom der Schweine (PRRSV) (Zugangsnummer KCTC 12096BP).

2. Impfzusammensetzung, umfassend ein Virus des koreanischen Reproduktions- und Atemwegssyndrom der Schweine (PRRSV) als einen effektiven Bestandteil.

3. Impfzusammensetzung nach Anspruch **2**, wobei das Virus durch Kultivieren des Virus während 3 bis 120 Durchgängen erhalten wird.

4. Impfzusammensetzung nach Anspruch **2** oder **3**, wobei die Impfzusammensetzung weiter Hilfsmittel, Füllstoffe oder Trägerstoffe umfasst.

5. Impfzusammensetzung nach einem der Ansprüche **2** bis **4**, wobei die Impfzusammensetzung weiter ein Schutzmittel umfasst.

6. Impfzusammensetzung nach Anspruch **5**, wobei das Schutzmittel Trehalose ist.

7. Virus des koreanischen Reproduktions- und Atemwegssyndrom der Schweine (PRRSV) (Zugangsnummer KCTC 12096BP) zur Verwendung bei der Vorbeugung des Reproduktions- und Atemwegssyndrom der Schweine.

8. PRRSV zur Verwendung nach Anspruch **7**, wobei das PRRSV intramuskulär oder intranasal inokuliert werden soll.

9. PRRSV zur Verwendung nach Anspruch **7** oder **8**, wobei das PRRSV mit einer Dosis von 2x10⁵ bis 2x10⁷ PFU/mL inokuliert werden soll.

10. Diagnosekit für ein Virus des koreanischen Reproduktions- und Atemwegssyndrom der Schweine, umfassend ein Virus des koreanischen Reproduktions- und Atemwegssyndrom der Schweine (Zugangsnummer KCTC 12096BP).

11. *In vitro*-Verfahren zum Nachweis eines Virus des koreanischen Reproduktions- und Atemwegssyndrom der Schweine, **gekennzeichnet durch** den Nachweis eines Virus des koreanischen Reproduktions- und Atemwegssyndrom der Schweine (Zugangsnummer KCTC 12096BP) in einer Zelle die infiziert werden soll oder infiziert ist, durch eine Antigen-Antikörper-Reaktion unter Verwendung eines Virus des koreanischen Reproduktions- und Atemwegssyndrom der Schweine (Zugangsnummer KCTC 12096BP).

12. *In vitro*-Verfahren nach Anspruch **11**, wobei die Analyse der Antigen-Antikörper-Reaktion unter Verwendung mindestens eines Verfahrens durchgeführt wird, ausgewählt aus der Gruppe, bestehend aus Gewebeimmunsfärbung, Radioimmunassay (RIA), Enzymimmunassay-Verfahren (ELISA), Western Blotting, Immunpräzipitationsassay, Immundiffusionsassay, Komplementbindungsassay, FACS und Protein-Chip.

## Revendications

1. Un virus du syndrome dysgénésique et respiratoire porcin (VSDRP) de type coréen (numéro d'accession KCTC 12096BP).

2. Une composition vaccinale comprenant un virus du syndrome dysgénésique et respiratoire porcin (VSDRP) de type coréen (numéro d'accession KCTC 12096BP) comme ingrédient actif.

3. La composition vaccinale selon la revendication **2**, dans laquelle le virus est obtenu en cultivant le virus pendant 3 à 120 passages.

4. La composition vaccinale selon la revendication **2** ou **3**, dans laquelle la composition vaccinale comprend en outre des adjuvants, des excipients ou des véhicules.

5. La composition vaccinale selon l'un quelconque des revendications **2** à **4**, dans laquelle la composition vaccinale comprend en outre un agent protecteur.

6. La composition vaccinale selon la revendication **5**, dans laquelle l'agent protecteur est le tréhalose.

7. Un virus du syndrome dysgénésique et respiratoire porcin (VSDRP) de type coréen (numéro d'accession KCTC 12096BP) pour son utilisation dans la prévention du syndrome dysgénésique et respiratoire porcin.

8. Le VSDRP pour son utilisation selon la revendication **7**, dans lequel le VSDRP sera inoculé par voie intramusculaire ou intranasale.

9. Le VSDRP pour son utilisation selon la revendication **7** ou **8**, dans lequel le VSDRP sera inoculé à une dose de 2x10⁵ à 2x10⁷ PFU/mL.

10. Un kit de diagnostic d'un virus du syndrome dysgénésique et respiratoire porcin de type coréen comprenant un virus du syndrome dysgénésique et respiratoire porcin (VSDRP) de type coréen (numéro d'accession KCTC 12096BP).

11. Une méthode *in vitro* pour détecter le virus du syndrome dysgénésique et respiratoire porcin de type coréen, **caractérisé par** la détection d'un virus du syndrome dysgénésique et respiratoire porcin de type coréen (numéro d'accession KCTC 12096BP) dans une cellule susceptible d'être infectée ou étant infectée, grâce à une réaction antigène-anticorps au moyen du virus du syndrome dysgénésique et respiratoire porcin de type coréen (numéro d'accession KCTC 12096BP).

12. La méthode *in vitro* selon la revendication **11**, dans laquelle l'analyse de la réaction antigène-anticorps est réalisée par le biais d'au moins une méthode sélectionnée dans un groupe consistant en la coloration immunitaire des tissus, le dosage radio-immunologique (RIA), le dosage immuno-enzymatique (ELISA), le Western Blotting, le test d'immunoprécipitation, le test d'immunodiffusion, le test de fixation du complément, le FACS, et la puce à protéine.
